Europäisches Patentamt

European Patent Office

Office européen des brevets

(11) Veröffentlichungsnummer: **0 325 112**
**A1**

(12) **EUROPÄISCHE PATENTANMELDUNG**

(21) Anmeldenummer: **89100117.4**

(22) Anmeldetag: **05.01.89**

(51) Int. Cl.⁴: **A61K 31/545 , A61K 9/14 , A61K 47/00**

(30) Priorität: **18.01.88 DE 3801179**

(43) Veröffentlichungstag der Anmeldung:
**26.07.89 Patentblatt 89/30**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Schwabe, Detlev, Dr.**
**Mailänder Strasse 16/163**
**D-6000 Frankfurt am Main(DE)**
Erfinder: **Leeb, Richard, Dr.**
**Nonnbornstrasse 15**
**D-6233 Kelkheim (Taunus)(DE)**
Erfinder: **Westenberger, Herbert**
**Sauerstrasse 19**
**D-6230 Frankfurt am Main 80(DE)**

(54) Stabilisierung von Cephalosporinderivaten durch Trocknung mit einem Stabilisator sowie stabile Zubereitungsformen mit Cephalosporinderivaten.

(57) Es werden stabile Zusammensetzung von Cephalosporinderivaten erhältlich durch Trocknung aus einer gemeinsamen Lösung eines Cephalosporinderivates als Wirkstoff und Laktose, Glucose, Saccharose oder Galaktose als Stabilisator sowie ein Verfahren zur Stabilisierung von Cephalosporinderivaten beschrieben.

EP 0 325 112 A1

## Stabilisierung von Cephalosporinderivaten durch Trocknung mit einem Stabilisator sowie stabile Zubereitungsformen mit Cephalosporinderivaten.

Es ist bekannt, daß Cephalosporinderivate häufig Stabilitätsprobleme haben. Oft kann die Stabilität durch Kristallisation begrenzt verbessert werden. Bei nicht kristallisierenden Cephalosporinderivaten wie Z.B. Cefpirom ist eine Stabilisierung auch durch vollständige Trocknung, Schutzbegasung oder Evakuierung bisher nicht möglich. Entsprechend den Angaben in der Europäischen Patentanmeldung mit der Veröffentlichungs-Nr. 0169700 soll der Zusatz von amorpher Laktose auf Ceftazidim einen stabilisierenden Effekt haben.

Untersuchungen haben gezeigt, daß der in der EP-A-0169700 beschriebene Effekt bei Mischung von Cefpirom mit amorpher Laktose nicht beobachtet wurde.

Es wurde überraschend gefunden, daß Laktose, Glucose, Saccharose oder Galaktose, insbesondere Laktose, nicht nur Cefpirom, sondern auch andere Cephalosporinderivate stabilisiert, wenn beide Substanzen aus gemeinsamer, wäßriger Lösung getrocknet werden, während ähnliche Substanzen wie z.B. Mannit, Sorbit, Fruktose und Cyclodextrinetherderivate die Zersetzung beschleunigen. Dies konnte auch deshalb nicht erwartet werden, weil die genannten Stabilisatoren weniger inert sind als z.B. Mannit oder Sorbit.

Die Erfindung betrifft daher ein Verfahren zur Stabilisierung von Cephalosporinderivaten, dadurch gekennzeichnet, daß man eine gemeinsame Lösung enthaltend ein Cephalosporinderivat als Wirkstoff und Laktose, Glucose, Saccharose oder Galaktose als Stabilisator trocknet, sowie durch Trocknung aus einer gemeinsamen Lösung eines Cephalosporinderivates als Wirkstoff und Laktose, Glucose, Saccharose oder Galaktose als Stabilisator erhaltene stabile Zusammensetzungen.

Die Zubereitungsform enthält einen oder mehrere der genannten Stabilisatoren.

Die gemeinsame Trocknung erfolgt nach üblichen Methoden wie z.B. Gefrier- oder Sprühtrocknung. Als Lösungsmittel für den Wirkstoff und den Stabilisator bietet sich insbesondere Wasser an.

Die Cephalosporinderivate liegen als freie Verbindungen oder als Salze vor. Je nach Wasserlöslichkeit und dem resultierenden pH-Wert der Lösung setzt man entweder die freie Verbindung oder ein physiologisch verträgliches Salz ein. Geeignete Cephalosporinderivate sind z.B. Cefotaxim, Cefpirom, oder auch 1-[-[(6R,7R)-[2-(2-Amino-4-thiazolyl)glyoxylamido]-2-carboxy-8-oxo-5-thia-1-azabicyclo [4.2.0]oct-2-en-3-yl]-methyl]-5,6,7,8-tetrahydrochinoliniumhydroxid, inneres Salz, $7^2$-(Z)-(0-methyloxim), sulfat (auch als HOE 111 bezeichnet). Im Falle von HOE 111 und Cefpirom wird also die freie Verbindung (Betain-Form) bzw. ein Säureadditionssalz und im Falle von Cefotaxim z.B. das Natriumsalz eingesetzt.

Die erfindungsgemäßen stabilen Zusammensetzungen eignen sich z.B. zur Herstellung von Injektionszubereitungen und zur Herstellung diagnostischer Präparate.

Die nachfolgenden Versuchsberichte/Beispiele erläutern die Erfindung:

EP 0 325 112 A1

**Versuchsbericht I**

Zubereitung: Die wäßrige Lösung von Cefpirom bzw. die gmeinsame Lösung von Cefpirom und Stabilisator oder Zusatzstoff wurde gefriergetrocknet.

| Zubereitung | Cefpirom 1g | Cefpirom 1g + Sorbit 1g | Cefpirom 1g + Fruktose 1g | Cefpirom 1g + Mannit 1g | Cefpirom 1g + Galaktose 1g | Cefpirom 1g + Saccharose 1g | Cefpirom 1g + Glucose 1g | Cefpirom 1g + Laktose 1g |
|---|---|---|---|---|---|---|---|---|
| nach Belastung 1 Monat + 40° C Wirkstoffgehalt in % von der unbelasteten Zubereitung | 62 % | 17 % | 47 % | 56 % | 75 % | 79 % | 80 % | 87 % |

Während Fruktose, Sorbit und Mannit eine Wirkstoffabnahme verursachen, wirken Galaktose, Saccharose, Glucose und Laktose stabilisierend, wobei Laktose den stärksten Effekt besitzt.

| Versuchsbericht II | | | |
|---|---|---|---|
| Die bloße trockene Zumischung amorpher Laktose zu getrocknetem Cefpirom (in Analogie zu den Angaben in EP-A-0169700) hat keinen stabilisierenden Effekt. | | | |
| Zubereitung | Cefpirom 1g wäßrige Lösung, gefriergetrocknet | Cefpirom 1g + Laktose 1g gemeinsame wäßrige Lösung gefriergetrokknet | Cefpirom 1g gefriergetrocknet + Laktose (amorph) 1g trocken zugemischt |
| Wirkstoffgehalt nach Belastung 7 Tage + 40° C in % von der unbelasteten Zubereitung | 80 % | 95 % | 73 % |

EP 0 325 112 A1

**Versuchsbericht III**

Das zur Stabilisierung optimale Mengenverhältnis von Laktose zu Cephalosporinderivat beträgt im Falle von Cefpirom 1:1.

| Zubereitung jeweils wäßrige Lösung gefriergetrocknet | Cefpirom 1g | Cefpirom 1g + Laktose 250 mg | Cefpirom 1g + Laktose 500 mg | Cefpirom 1g + Laktose 1000mg | Cefpirom 1g + Laktose 1500 mg |
|---|---|---|---|---|---|
| Wirkstoffgehalt nach Belastung 3 Monate + Raumtemp. in % von der unbelasteten Zubereitung | 87 % | 91 % | 92 % | 95 % | 95 % |

EP 0 325 112 A1

**Versuchsbericht IV**

Durch einen weiteren Glycinzusatz ist eine Verbesserung des Stabilisierungseffektes möglich; Glycin alleine ohne Laktose zeigt keinen Stabilisierungseffekt

| Zubereitung wäßrige Lösung gefriergetrocknet | Cefpirom 1g + Laktose 1g | Cefpirom 1g + Glycin 1g | Cefpirom 1g + Glycin 1g + Laktose 1g |
|---|---|---|---|
| Wirkstoffgehalt nach Belastung 7 Tage + 40°C in % von der unbelasteten Zubereitung | 95 % | 81 % | 96 % |

| Zubereitung wäßrige Lösung gefriergetrocknet | Cefpirom 1g + Laktose 1g | Cefpirom 1g + Glycin 1g | Cefpirom 1g + Glycin 1g + Laktose 1g |
|---|---|---|---|
| Wirkstoffgehalt nach Belastung 1 Monat + 40°C in % von der unbelasteten Zubereitung | 87 % | - | 92 % |

6

| Versuchsbericht V | | |
|---|---|---|
| Der Stabilisierungseffekt durch Laktose wird nicht durch die Art der Trocknung der gemeinsamen Lösung beeinflußt. | | |
| Zubereitung | Cefpirom/Laktose 1:1, sprühgetrocknet aus wäßriger Lösung | Cefpirom sprühgetrocknet aus wäßriger Lösung |
| Belastung: | 2 Monate Lagerung + 40°C | 2 Monate Lagerung +40°C |
| Gehaltsverlust durch Zersetzung | 19 % | 36 % |
| Klarlöslichkeit (Wahrnehmung mit dem bloßen Auge) (nach Lösung in $H_2O$) | klar | trüb |
| Farbe der Lösung (Wahrnehmung mit dem bloßen Auge) (nach Lösung in $H_2O$) | hell | teebraun |

| Versuchsbericht VI | | |
|---|---|---|
| Stabilisierung weiterer Cephalosporinderivate mit Laktose | | |
| Zubereitung wäßrige Lösung gefriergetrocknet | HOE 111 1g | HOE 111 1g + Laktose 1g |
| Wirkstoffgehalt nach Belastung 3 Monate + Raumtemp. in % von der unbelasteten Zubereitung | 82 % | 92 % |
| Zubereitung wäßrige Lösung gefriergetrocknet | Cefotaxim-Natrium 1g | Cefotaxim-Natrium 1g + Laktose 1g |
| Wirkstoffgehalt nach Belastung 3 Monate + 40°C in % von der unbelasteten Zubereitung | 72 % | 80 % |

## Ansprüche

1. Verfahren zur Stabilisierung von Cephalosporinderivaten, dadurch gekennzeichnet, daß man eine gemeinsame Lösung enthaltend ein Cephalosporinderivat als Wirkstoff und Laktose, Glucose, Saccharose oder Galaktose als Stabilisator nach üblichen Methoden trocknet.

2. Verfahren gemäß Anspruch 1, dadurch gekennzeichnet, daß Laktose als Stabilisator verwendet wird.

3. Verfahren gemäß Anspruch 1 oder 2, dadurch gekennzeichnet, daß als Wirkstoff Cefpirom, HOE 111 oder Cefotaxim-Natrium verwendet wird.

4. Verfahren gemäß einem oder mehreren der Ansprüche 1 bis 3, dadurch gekennzeichnet, daß das Mengenverhältnis (in g) von Wirkstoff zu Stabilisator 1:1 beträgt.

5. Verfahren gemäß einem oder mehreren der Ansprüche 1 bis 4, dadurch gekennzeichnet, daß die Trocknung eine Gefriertrocknung oder Sprühtrocknung ist.

6. Verfahren gemäß einem oder mehreren der Ansprüche 1 bis 5, dadurch gekennzeichnet, daß zusätzlich Glycin verwendet wird.

7. Stabile Zusammensetzung von Cephalosporinderivaten erhältlich nach Verfahren gemäß Anspruch 1 durch Trocknung aus einer gemeinsamen Lösung eines Cephalosporinderivates als Wirkstoff und Laktose, Glucose, Saccharose oder Galaktose als Stabilisator.

8. Stabile Zusammensetzung gemäß Anspruch 7, dadurch gekennzeichnet, daß sie einen oder mehrere Stabilisatoren enthält.

9. Stabile Zusammensetzung gemäß Anspruch 7, dadurch gekennzeichnet, daß eine oder mehrere der folgenden Bedingungen zutrifft:
der Wirkstoff ist Cefpirom, HOE 111 oder Cefotaxim-Natrium,
der Stabilisator ist Laktose,
das Gewichtsverhältnis von Wirkstoff zu Stabilisator ist 1 :1
Glycin ist als zusätzlicher Stabilisator enthalten und Die Trocknung ist eine Gefrier- oder Sprühtrocknung.

**Europäisches Patentamt**

# EUROPÄISCHER RECHERCHENBERICHT

Nummer der Anmeldung

EP 89 10 0117

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| P,X | EP-A-0 253 329  (EISAI CO., LTD)<br>* Seite 1, Zeilen 1-25; Seite 2, Zeilen 1-28 *<br>--- | 1,2,4,5,7,8 | A 61 K  31/545<br>A 61 K   9/14<br>A 61 K  47/00 |
| X | EP-A-0 134 568  (SHIONOGI & CO., LTD)<br>* Seite 1, Zeilen 1-11; Seite 2, Zeile 19 - Seite 4, Zeile 6; Ansprüche 1,5,9,10 *<br>--- | 1,5,7,8 | |
| X | GB-A-2 078 737  (SHIONOGI & CO., LTD)<br>* Seite 1, Zeilen 9-17,37-42; Seite 2, Zeilen 3-16; Ansprüche 1,8,10,11,19 * | 1,4,5,7,8 | |
| Y | | 2,3,6,9 | |
| | --- | | |
| D,Y | EP-A-0 169 700  (ELI LILLY AND CO.)<br>* Seite 2, Zeilen 1-11; Seite 4, Zeile 8 - Seite 5, Zeile 13 *<br>--- | 2 | |
| Y | CHEMICAL ABSTRACTS, Band 99, Nr. 25, 19. Dezember 1983, Seiten 394-395, Zusammenfassung Nr. 209684q, Columbus, Ohio, US; K. MACHKA et al.: "In vitro activity of HR 810, a new broad-spectrum cephalosporin", & EUR. J. CLIN. MICROBIOL. 1983, 2(4), 345-9<br>* Zusammenfassung *<br>--- | 3,9 | RECHERCHIERTE SACHGEBIETE (Int. Cl.4)<br><br>A 61 K |
| Y | CHEMICAL ABSTRACTS, Band 86, Nr. 2, 10. Januar 1977, Seite 197, Zusammenfassung Nr. 8620y, Columbus, Ohio, US; & JP-A-76 32 723 (TOYAMA CHEMICAL CO., LTD) 19-03-1976<br>* Zusammenfassung *<br>--- | 6 | |
| A | DE-A-2 017 373  (ORSYMONDE S.A.)<br>* Seiten 5,6 *<br>----- | 2,6 | |

Der vorliegende Recherchenbericht wurde für alle Patentansprüche erstellt

| Recherchenort | Abschlußdatum der Recherche | Prüfer |
|---|---|---|
| DEN HAAG | 24-04-1989 | MUELLNERS W. |